# EUROPÄISCHE PATENTSCHRIFT

(11) **EP 0 610 357 B1**
(45) Veröffentlichungstag und Bekanntmachung des Hinweises auf die Patenterteilung: **17.09.1997**
(21) Anmeldenummer: 92922822.9
(22) Anmeldetag: 21.10.1992
(51) Int. Cl.: A61K 9/70

(54) **TRANSDERMALE THERAPEUTISCHE SYSTEME MIT KRISTALLISATIONSINHIBITOREN**
TRANSDERMAL THERAPEUTIC SYSTEMS CONTAINING CRYSTALLIZATION INHIBITORS
SYSTEMES TRANSDERMIQUES THERAPEUTIQUES CONTENANT DES INHIBITEURS DE CRISTALLISATION

(30) Priorität: 31.10.1991 DE 4136057; 27.03.1992 DE 4210711
(43) Veröffentlichungstag der Anmeldung: 17.08.1994
(73) Patentinhaber: SCHERING AKTIENGESELLSCHAFT, 13353 Berlin (DE)
(72) Erfinder: LIPP, Ralph, D-10717 Berlin (DE); RIEDL, Jutta, D-14057 Berlin (DE); TACK, Johannes, D-13595 Berlin (DE)
(86) Internationale Anmeldenummer: EP9202478
(87) Internationale Veröffentlichungsnummer: WO9308795

(56) Entgegenhaltungen:
- EP-A- 0 208 395
- EP-A- 0 289 977
- EP-A- 0 421 454
- EP-A- 0 450 986
- WO-A-91/05529
- DATABASE WPIL Section Ch, Week 8406, 27. Dezember 1983 Derwent Publications Ltd., London, GB; Class A12, AN 84-034222
- DATABASE WPI Section Ch, Week 7934, 14. Juli 1979 Derwent Publications Ltd., London, GB; Class A12, AN 79-62497B
- DATABASE WPIL Section Ch, Week 9151, 11. November 1991 Derwent Publications Ltd., London, GB; Class A04, AN 91-373554
- NEUMULLER O.A. 'Römpps Chemie lexikon' 1983 , FRANKSCH VERLAG , STUTTGART ,DE

## Beschreibung

Die Erfindung betrifft transdermale therapeutische Systeme, die dem Organismus über die Haut Wirkstoffe verfügbar machen und dadurch gekennzeichnet sind, daß in der wirkstoffhaltigen Matrix Kristallisationsinhibitoren enthalten sind.

Transdermale therapeutische Systeme (TDS) sind bekanntlich mehrschichtig aufgebaute Pflaster, die auf der Haut fixiert werden und die den Wirkstoff perkutan über einen längeren Zeitraum kontinuierlich abgeben. Im wesentlichen bestehen transdermale therapeutische Systeme aus einer für Wasser, Penetrationsverstärker und Wirkstoffe impermeablen Abdeckfolie, einer Matrix, die den Hauthaftklebstoff, Penetrationsverstärker und Arzneistoff umfaßt und einer ablösbaren Schutzfolie.

Hohe Konzentrationen an gelöstem Wirkstoff in der Matrix transdermaler therapeutischer Systeme ermöglichen in der Regel einen hohen Wirkstofffluß durch die Haut. Insbesondere wird in jüngerer Zeit häufig von sogenannten übersättigten Systemen berichtet, die den gewünschten hohen transdermalen Fluß von Arzneistoffen ermöglichen (K. H. Ziller und H. H. Rupprecht, Pharm. Ind. 52, Nr. 8 (1990), 1017-1022).

Ein Problem solcher übersättigter Lösungen ist die ungenügende Lagerstabilität. Denn handelt es sich bei den eingearbeiteten Wirkstoffen um gut kristallisierende Verbindungen, so muß mit Kristallisationsvorgängen während der Lagerung gerechnet werden. Diese Neigung zur Kristallbildung respektive zum Kristallwachstum ist beispielsweise bei Suspensionen und übersättigten Lösungen von Steroidhormonen bekannt (M. Kuhnert-Brandstätter et al., Sci. Pharm., 35 (1967) 4, 287-297). Dieses Phänomen trifft auch auf übersättigte Lösungen schwer löslicher Stoffe in Acrylatkleber-Enhancergemischen zu.

Auf Grund des Kristallisationsvorgangs verschiebt sich der Anteil von gelöstem zu kristallisiertem Wirkstoff. Hierbei kann gegebenenfalls sogar die Sättigungskonzentration des Wirkstoffs im System unterschritten werden (Jian-wei Yu et al., Drug Development and Industrial Pharmacy 17, 1991, 1883 ff). Zusätzlich führt Kristallwachstum zur Reduktion von Kristalloberfläche, wodurch die Lösungsgeschwindigkeit während der Applikation herabgesetzt wird.

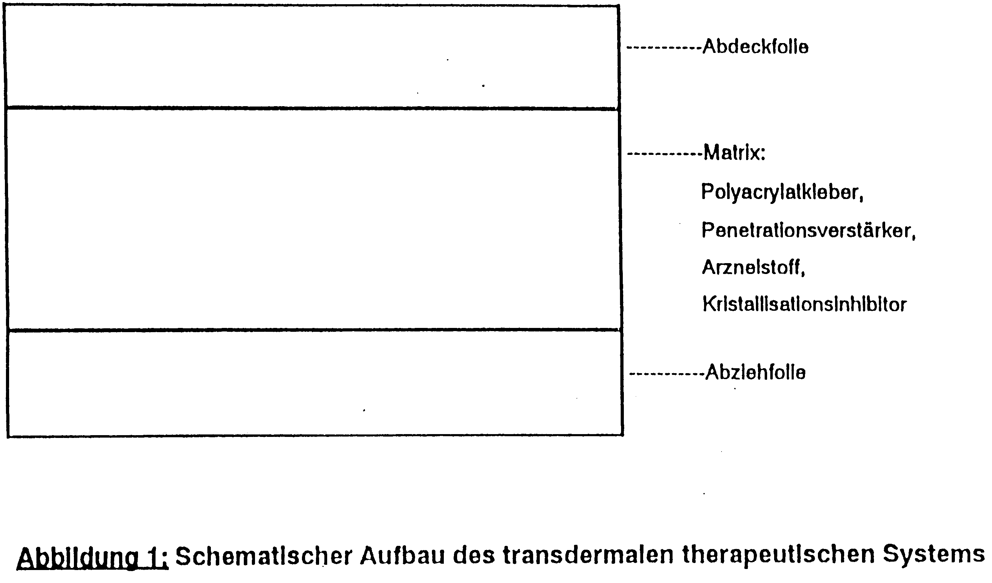

Um Kristallisationsvorgänge in transdermalen therapeutischen Systemen zu verhindern und die therapeutisch erwünschte Dosis kontinuierlich applizieren zu können, werden erfindungsgemäß Kristallisationsinhibitoren zugesetzt (Abb. 1). Durch den Zusatz von Kristallisationsinhibitoren bleibt ein hoher Anteil an Wirkstoff während der Lagerzeit gelöst. Die dadurch erreichte physikalische Stabilität der erhaltenen Transdermalsysteme ist Grundvoraussetzung für die Anwendung in der Praxis. Transdermale therapeutische Systeme, in die erfindungsgemäß Kristallisationsinhibitoren eingearbeitet sind, zeichnen sich durch sehr gute in vitro-Wirkstoffabgabe aus - wie Abbildung 2 am Beispiel von 17β-Estradiol zeigt. Gleichzeitig werden lagerbedingte Kristallisationsprozesse der Wirkstoffe in den erfindungsgemäßen TDS verhindert (Tab. 1). Sie sind daher in besonderem Maße geeignet, den Wirkstoff am Menschen in therapeutisch relevanten Dosen kontinuierlich bioverfügbar zu machen. So zeigte beispielsweise ein 17β-Estradiol-TDS in Anwesenheit eines Kristallisationsinhibitors wie Siliciumdioxid deutlich geringere Neigung zur Kristallbildung auf als ein Vergleichs-TDS ohne Siliciumdioxid-Zusatz. Während in dem erfindungsgemäßen System über den Beobachtungszeitraum von 8 Monaten bei Raumtemperaturlagerung kein Kristallwachstum festgestellt wurde, haben sich in dem System ohne Kristallisationsinhibitoren große Kristalle (- 730 µm) gebildet (Tab. 1). Als Kristallisationsinhibitoren sind hochdisperses Siliciumdioxid oder makromolekulare Stoffe geeigent. Als makromolekulare Stoffe seien beispielsweise Polyvinylpyrrolidone mit einem mittleren Molekulargewicht von etwa 1 000 bis 2 000000 (beispielsweise Kollidon® 12 PF, Kollidon® 17 PF, Kollidon® 25 PF, Kollidon® 30,, Kollidon® 90 der Firma BASF, Vinylpyrrolidon-Vinylacetat-Copolymere (wie das Kollidon® VA 64 der Firma BASF), quervernetzte Polyvinylpyrrolidone (wie das Kollidon® CL der Firma BASF), Polyvinylalkohol, Hydroxypropylcellulose, Ethylcellulose, Gelatine, Stärke(derivate), Dextrine und Dextrane, wie beispielsweise α-, β-, und γ-Cyclodextrin, Dimethyl-β-cyclodextrin und 2-Hydroxypropyl-β-cyclodextrin), Sterine (wie Cholesterol) oder Gallensäuren (wie Cholsäure oder Lithocholsäure) genannt.

Hierbei zeichnen sich insbesondere die Polyvinylpyrrolidone, deren Copolymerisate mit Vinylacetat und hochdisperses Siliciumdioxid durch eine hohe kristallisationsinhibitorische Potenz aus.

Kristallisationsinhibitoren können in allen bekannten transdermalen Systemen verwendet werden, wie beispielsweise in Polyacrylatsystemen oder in Systemen auf Basis von Silikon- oder Synthesekautschuk-Hauthaftklebstoffen, wobei der Inhibitor in Konzentrationen von 0,1 bis 40 Gewichtsprozenten bezogen auf das Gesamtgewicht der Matrix eingearbeitet wird. Zusätzlich zu Hauthaftklebstoff, Wirkstoff und Kristallisationsinhibitor kann die Matrix gewünschtenfalls Penetrationsverstärker enthalten, wobei alle bekannten Penetrationsverstärker und deren Gemische in den üblichen Konzentrationen eingesetzt werden.

Als Penetrationsverstärker sind beispielsweise geeignet:
Ein- und mehrwertige Alkohole mit bis zu 24 Kohlenstoffatomen, wie 1,2-Propandiol, 1,3-Propandiol, 1,2-Ethandiol, Glycerin oder Laurylalkohol; freie Carbonsäuren mit bis zu 24 Kohlenstoffatomen, wie Laurinsäure; Fettsäureester mit bis 24 Kohlenstoffatomen in der Fettsäurekomponente und bis zu 20 Kohlenstoffatomen in der ein- oder mehrwertigen Alkoholkomponente, wie Isopropylmyristat, Glycerinmonopalmitat, Dodecanoylacetat; Terpene, Amide Harnstoff und Gemische dieser Penetrationsverstärker.

Die Konzentrationen der Penetrationsverstärker bzw. der Gemische der genannten Stoffklassen können zwischen 0,5 bis 40 Gewichtsprozenten bezogen auf das Gesamtgewicht der Matrix liegen.

Bevorzugte Konzentrationsbereiche sind für 1,2-Propandiol 15-25 Gewichtsprozent, für Fettsäureester, freie Carbonsäuren und Alkohole mit 8-24 Kohlenstoffatomen 0,5-15 Gewichtsprozent und Enhancermischungen, die in Mischungsverhältnissen von 1:10 bis 10:1 möglich sind, beispielsweise für 1,2-Propandiol und Laurinsäure, 5-40 Gewichtsprozent, vorzugsweise 20-30 Gewichtsprozent bezogen auf die fertige Matrix.

Wirkstoffe, die sich zur Herstellung der erfindungsgemäßen transdermalen Systeme eignen sind vorzugsweise solche, die in üblichen Klebersystemen schwer löslich oder unlöslich sind und gut kristallisieren, wie beispielsweise Steroidhormone wie: Gestagen wirksame Steroidhormone, wie beispielsweise das 13-Ethyl-17β-hydroxy-18,19-dinor-17α-pregn-4-en-20yl-3-on (=Levonorgestrel), das 13-Ethyl-17β-hydroxy-18,19-dinor-17α-pregna-4,15-dien-20yn-3-on (=Gestoden), das 13-Ethyl-17β-hydroxy-11-methylen-18,19-dinor-17α-pregn-4-en-20yn (=Desorgestrel) oder das 13-Ethyl-11-methylen-17β-hydroxy-18,19-dinor-17α-pregn-4-en-3-on (3-Keto-Desogestrel) Estrogen wirksame Steroidhormone das 3-Hydroxy-1,3,5-(10)-estratrien-17-on (=Estron), das 1,3,5(10)-Estratrien-3,17β-diol (=Estradiol) oder das 1,9-Nor-17α-pregna-1,3,5(10)-trien-20yn-3,17β-diol (=Ethinylestradiol), das 17β-Hydroxy-19-nor-17α-pregn-4en-20yn-3-on (=Norethisteronacetat), das 14α,17α-Ethano-1,3,5(10)-estratrien-3,17β-diol (=Cyclodiol) und das 14α,17α-Ethano-1,3,5(10)-estratrien-3,16α,17β-triol (=Cyclotriol) und Kombinationen dieser Gestagene und Estrogene.

Androgen wirksame Steroidhormone wie das 17β-Hydroxy-4-androsten-3-on (=Testosteron) und dessen Ester oder das 17β-Hydroxy-1α-methyl-5α-androsten-3-on (=Mesterolon).

Antiandrogen wirksame Steroidhormone wie das 17α-Acetoxy-6-chlor-1β,2β-dihydro-3H-cyclopropa[1,2]-pregna-1,4,6-trien-3,20-dion (=Cypoteronacetat).

Kortikoide wie das 11β,17α,21-Trihydroxy-4-pregnen-3,20-dion (=Hydrocortison), das 11β,17α,21-Trihydroxy-1,4-pegnadien-3,20-dion (=Prednisolon), das 11β,17α,21-Trihydroxy-6α-methyl-1,4-pregnatrien-3,20-dion (=Methylprednisolon) und das 6α-Fluor-11β,21-dihydroxy-16α-methyl-1,4-pregnadien-3,20-dion (=Diflucortolon) und deren Ester.

Geeignete Wirkstoffe sind ferner:
Ergolin-Derivate, wie das Lisurid, [=3-(9,10-Didehydro-6-methyl-8α-ergolinyl)-1,1-diethylharnstoff],das Bromlisurid [=3-(2-Brom-9,10-dehydro-6-methyl-8α-ergolinyl-1,1-diethylharnstoff] das Tergurid [=3-(6-Methyl-8α-ergolinyl-1,1-diethylharnstoff] und das Protergurid [=3-(6-Propyl-8α-ergolinyl)-1,1-diethylharnstoff].

Antihypertonika wie das 7α-Acetylthio-17α-hydroxy-3-oxo-4-pregnen-21-carbonsäure-γ-lacton (=Spironolacton) und das 7α-Acetylthio-15β-,16β-methylen-3-oxo-17α-pregna-1,4-dien-21,17-carbolacton (=Mespirenon).

Antikoagulantia wie die 5-[Hexahydro-5-hydroxy-4-(3-hydroxy-4-methyl-1-octen-6-ynyl)-2(1H)-pentalenyliden)]-pentansäure (=Iloprost) oder die (Z)-7-[(1R,2R,3R,5R)-5-Chlor-3-hydroxy-2-[(E)-(3R)-3-hydroxy-4,4-dimethyl-1-octenyl]-cyclopentyl]-5-heptensäure (=Nocloprost).

Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on (=Diazepam).

Organische Nitroverbindungen, wie das Isosorbiddinitrat [=1,4,3,6-Dianhydro-D-glucitol-dinitrat].

Betablocker, wie das Propanolol {= 1-[(1-Methylethyl)-amino]-3-(1-naphthyloxy-2-propanolol}, das Mepindolol {= 1-[(1-Methylethyl)-amino]-3-[(2-methyl-1H-inol-4-yl)-oxy]-2-propanol} und das Carazolol {= 2-(9H-Carbazol-4-yloxy)-3-[(1-methethyl)-amino]-2-propanol}.

Carotinoide wie das α-Carotin und das β-Carotin.

Eine weitere Gruppe sind β-Carboline, wie der 5-Isopropyl-4-methyl-β-carbolin-3-carbonsäure-ethylester und der 5-Isopropyl-4-methoxymethyl-β-carbolin-3-carbonsäureethylester und weitere β-Carboline, die in den Europäischen Patentanmeldungen 234,173 und 239,667 beschrieben sind. Erwähnenswert sind auch hochwirksame Analgetika, wie zum Beispiel das 7,8-Didehydro-4,5-epoxy-17-methylmorphinan-3,6-diol (=Morphin), das 4,5-Epoxy-14-hydroxy-3-methoxy-17-methylmorphinan-6-on (=Oxycodon), das (-)-(R)-6-(Dimethylaminol-4,4-diphenyl-3-heptanon (=Levomethadon) oder das 3,4,5,6-Tetrahydro-5-methyl-1-phenyl-1H-2,5-benzoxacin (=Nefopam).

Letztlich sei als geeigneter Wirkstoff das Scopolamin erwähnt.

Es ist einleuchtend, daß die erfindungsgemäßen transdermalen Systeme auch Gemische dieser Wirkstoffe enthalten können.

Die optimale Konzentration des Wirkstoffs in den erfindungsgemäßen transdermalen therapeutischen Systemen ist naturgemäß von der Art des Wirkstoffs, seiner Wirksamkeit, der Art der Penetrationsverstärker, des verwendeten Klebers etc. abhängig und muß im Einzelfalle durch die dem Galeniker vertrauten Vorversuche ermittelt werden. In der Regel wird er den Wirkstoff so dosieren, daß seine Konzentration in der fertigen Matrix 0,1 bis 10 Gewichtsprozent bezogen auf diese beträgt.

Die erfindungsgemäßen transdermalen therapeutischen Systeme sind vorzugsweise so beschaffen, daß sie aus einer für die Penetrationsverstärker und gegebenenfalls auch für Wasser undurchlässigen Deckschicht, einer auf der Deckschicht haftenden wirkstoffhaltigen Klebematrix, welche Kristallisationsinhibitor und Penetrationsverstärker enthält und einer abziehbaren Schutzschicht bestehen.

Diese einfachste Form eines transdermalen therapeutischen Systems, kann in der Weise hergestellt werden, daß man eine Lösung des Klebers in einem niedrigsiedenden Lösungsmittel mit dem Wirkstoff oder Wirkstoffgemisch, dem Penetrationsverstärker und dem Kristallisationsinhibitor mischt, die Mischung folienartig auf einer undurchlässigen abziehbaren Schutzschicht aufträgt, das flüchtige Lösungsmittel durch Erwärmen entfernt und das erhaltene Produkt mit einer Deckschicht abdeckt.

Geeignete Lösungsmittel zur Auflösung des Klebers sind beispielsweise niedrigsiedende Alkohole, wie Methanol, Ethanol oder Isopropanol, niedrigsiedende Ketone, wie Aceton, niedrigsiedende Kohlenwasserstoffe wie Hexan, oder niedrigsiedende Ester, wie Ethylacetat sowie Mischungen derselben.

Dieses Verfahren kann in der Weise durchgeführt werden, daß eine Lösung oder Suspension des Wirkstoffs, des Kristallisationsinhibitors, der Penetrationsverstärker und des Klebers in einem flüchtigen Lösungsmittel auf eine abziehbare Schutzschicht gestrichen und nach dem Trocknen bei etwa 60° C bis 90° C mit einer planen undurchlässigen Deckschicht versehen wird.

Als abziehbare Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 µm dicke Folien aus PVC, PVDC oder deren Kopolymerisate EVA, Polyethylen oder Polyester sowie deren Koextrudate wahlweise transparent pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Fläche von 5 bis 100 cm².

Es ist für den Fachmann offenkundig, daß die erfindungsgemäßen transdermalen therapeutischen Systeme auch wesentlich komplexer gestaltet werden können als die bereits erwähnten einfachen Matrixsysteme (Yie W. Chien: "Transdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Research 1030 Hamilton Court Menlo Park CA 94025 (415) 328-2228). Dies dürfte aber in der Regel keinerlei nennenswerte Vorzüge der Systeme bringen, die den erhöhten Aufwand zu ihrer Herstellung rechtfertigen.

Die nachfolgenden Ausführungsbeispiele dienen zur näheren Erläuterung der Erfindung:

### Beispiel 1

### Transdermales therapeutisches System mit 17β-Estradiol (3,3 mg/10 cm²)

Zu 122 g einer 50 gewichtsprozentigen Lösung von Polyacrylat-Hauthaftklebstoff Gelva 2723 (Hersteller: Monsanto Chemical Company, Springfield, Massachusetts) werden nacheinander
- 3,00 g: 17β-Estradiol
- 35,00 g: 1,2-Propandiol und
- 1,0 g: Siliciumdioxid, hochdisperses
(z.B. Aerosil 200 der Degussa AG, Frankfurt/M.,BRD)
gegeben. Die sich bildende trübe Masse wird anschließend in einem Edelstahlgefäß gerollt, um eine Blasenbildung während des Mischens gering zu halten.

Mittels einer Knife-over-Roll Beschichtungsvorrichtung wird die weitgehend gasblasenfreie Masse auf eine silikonisierte Polyesterfolie (Abziehfolie: z.B. FDA-PET release liner) in der Weise aufgetragen, daß nach dem Entfernen des flüchtigen Lösungsmittels (Ethylacetat) bei 65-75° C über 2 bis 3 Minuten ein gleichmäßiger Film von 100 g/m² entsteht. Anschließend wird mit einer PVDC-Abdeckfolie (Saran 18L, 30 µm der Firma Dow Chemical, Midland, MI, USA) kaschiert. Das so erhaltene Laminat wird mittels einer Stanzvorrichtung in Einzelpflaster von 2,5 cm² - 25 cm², vorzugsweise 10 cm² Fläche geteilt und in aluminierte Beutel verpackt. Die Pflaster haften nach Abziehen der Schutzfolie auf der Haut und können zur Hormonsubstitution verwendet werden.

### Beispiel 2

### Transdermales therapeutisches System mit 17β-Estradiol (3,3 mg/10 cm²)

Zu 122 g einer 50 gewichtsprozentigen Lösung von Polyacrylat-Hauthaftklebstoff Gelva 2723 (Hersteller: Monsanto Chemical Company, Springfield, Massachusetts) werden nacheinander
- 3,00 g: 17β-Estradiol
- 35,00 g: 1,2-Propandiol und
- 1,0 g: Cholesterol
gegeben. Die sich bildende trübe Masse wird anschließend in einem Edelstahlgefäß gerollt, um eine Blasenbildung während des Mischens gering zu halten.

Mittels einer Knife-over-Roll Beschichtungsvorrichtung wird die weitgehend gasblasenfreie Masse auf eine silikonisierte Polyesterfolie (Abziehfolie: z.B. FDA-PET release liner) in der Weise aufgetragen, daß nach dem Entfernen des flüchtigen Lösungsmittels (Ethylacetat) bei 65-75° C über 2 bis 3 Minuten ein gleichmäßiger Film von 100 g/m² entsteht. Anschließend wird mit einer PVDC-Abdeckfolie (Saran 18L, 30 µm der Firma Dow Chemical, Midland, MI, USA) kaschiert. Das so erhaltene Laminat wird mittels einer Stanzvorrichtung in Einzelpflaster von 2,5 cm² - 25 cm², vorzugsweise 10 cm² Fläche geteilt und in aluminierte Beutel verpackt. Die Pflaster haften nach Abziehen der Schutzfolie auf der Haut und können zur Hormonsubstitution verwendet werden.

### Beispiel 3

### Transdermales therapeutisches System mit 17β-Estradiol

- 2,00 g: 17β-Estradiol
- 5,00 g: Isopropylmyristat und
- 10,00 g: Kollidon ®VA 64
werden in 20 g Isopropanol gelöst und zu 166 g Gelva ®2723 (50%ige Lösung in Ethylacetat) gegeben. Die sich bildende trübe Masse wird anschließend in einem Edelstahlgefäß gerollt, um eine Blasenbildung gering zu halten.

Die Herstellung der Pflaster erfolgt wie im Beispiel 1 beschrieben.

### Beispiel 4

### Transdermales therapeutisches System mit 17β-Estradiol

- 4,00 g: 17β-Estradiol
- 12,00 g: Kollidon ®12 PF und
- 35,00 g: 1,2-Propandiol
werden in 20 g Isopropanol gelöst und zu 98 g Gelva ®2723 (50%ige Lösung in Ethylacetat) gegeben. Die sich bildende trübe Masse wird anschließend in einem Edelstahlgefäß gerollt, um eine Blasenbildung gering zu halten.

Die Herstellung der Pflaster erfolgt wie im Beispiel 1 beschrieben.

### Beispiel 5

### Transdermales therapeutisches System mit Gestoden

- 2,00 g: Gestoden
- 5,00 g: Isopropylmyristat und
- 10,00 g: Kollidon ®VA 64
werden in 20 g Isopropanol gelöst und zu 166 g Gelva ®2723 (50%ige Lösung in Ethylacetat) gegeben. Die sich bildende trübe Masse wird anschließend in einem Edelstahlgefäß gerollt, um eine Blasenbildung gering zu halten.

Die Herstellung der Pflaster erfolgt wie im Beispiel 1 beschrieben.

### Beispiel 6

### Transdermales therapeutisches System mit Gestoden

- 4,00 g: Gestoden
- 12,00 g: Kollidon ®12 PF und
- 35,00 g: 1,2-Propandiol
werden in 20 g Isopropanol gelöst und zu 98 g Gelva ®2723 (50%ige Lösung in Ethylacetat) gegeben. Die sich bildende trübe Masse wird anschließend in einem Edelstahlgefäß gerollt, um eine Blasenbildung gering zu halten.

Die Herstellung der Pflaster erfolgt wie im Beispiel 1 beschrieben.

### Beispiel 7

### Transdermales therapeutisches System mit Levonorgestrel

- 2,00 g: Levonorgestrel
- 5,00 g: Isopropylmyristat und
- 10,00 g: Kollidon ®VA 64
werden in 20 g Isopropanol gelöst und zu 166 g Gelva ®2723 (50%ige Lösung in Ethylacetat) gegeben. Die sich bildende trübe Masse wird anschließend in einem Edelstahlgefäß gerollt, um eine Blasenbildung gering zu halten.

Die Herstellung der Pflaster erfolgt wie im Beispiel 1 beschrieben.

**Tabelle 1**

| **Anwesenheit von Wirkstoffkristallen in 17-β-Estradiol-TDS mit und ohne SiO**_{**2**} **als Kristallisationsinhibitor** | | | |
|---|---|---|---|
| Zusammensetzung der TDS-Matrizes (pro 100 mg) | Maximale Kristallgröße nach RT-Lagerung über | | |
| | 1 Monat | 3 Monate | 8 Monate |
| 71,6 mg Acrylatkleber, 24 mg 1,2-Propandiol, 3,3 mg E₂, 1,1 mg SiO₂, = Beispiel1 | < 6 µm | ohne Kristalle | ohne Kristalle |
| 72,7 mg Acrlyatkleber, 24 mg 1,2-Propandiol, 3,3 mg E₂ | - 450 µm | - 400 µm | - 730 µm |

## Patentansprüche

1. Transdermales therapeutisches System, dadurch gekennzeichnet, daß es
a) aus einer für Wasser, Penetrationsverstärker und Wirkstoffundurchlässigen Deckschicht,
b) einer auf der Deckschicht haftenden Klebermatrix enthaltend
b1) einen oder mehrere in üblichen Klebersystemen schwer löslichen oder unlöslichen, gut kristallisierenden Wirkstoff,
b2) ein Vinylpyrrolidon-Vinylacetat-Copolymer als Kristallisationsinhibitor,
b3) gegebenenfalls einen Penetrationsverstärker,
c) einer abziehbaren Schutzschicht
besteht.

2. Transdermales therapeutisches System nach Anspruch 1, dadurch gekennzeichnet, daß der Wirkstoff b1) ein Steroidhormon ist.

## Claims

1. Transdermal therapeutic system, characterised in that it consists of
a) a cover layer that is impermeable to water, to penetration enhancer and to active ingredient,
b) an adhesive matrix that adheres to the cover layer and comprises
b1) one or more active ingredient(s) that crystallise(s) readily and is/are insoluble or sparingly soluble in customary adhesive systems,
b2) a vinylpyrrolidone/vinyl acetate copolymer as crystallisation inhibitor,
b3) optionally a penetration enhancer,
c) a removable protective layer.

2. Transdermal therapeutic system according to claim 1, characterised in that the active ingredient b1) is a steroid hormone.

## Revendications

1. Système thérapeutique transdermique caractérisé en ce qu'il est constitué
a) d'une couche de couverture imperméable à l'eau, à l'activateur de pénétration et au principe actif,
b) d'une matrice adhésive adhérente à la couche de couverture, laquelle matrice contient
b1) un ou plusieurs principes actifs aisément cristallisables peu solubles ou insolubles dans les systèmes d'adhésifs usuels,
b2) un copolymère de vinylpyrrolidone-acétate de vinyle en tant qu'inhibiteur de cristallisation,
b3) éventuellement un activateur de pénétration,
c) d'une couche protectrice amovible.

2. Système thérapeutique transdermique selon la revendication 1, caractérisé en ce que le principe actif b1) est une hormone stéroïde.
